# EUROPEAN PATENT SPECIFICATION

(11) **EP 4 416 423 B1**
(45) Date of publication and mention of the grant of the patent: **24.09.2025**
(21) Application number: 22805783.2
(22) Date of filing: 10.10.2022
(51) Int. Cl.: F21V 7/04, F21V 11/02, F21V 8/00

(54) **A LIGHT EMITTING DEVICE**
LICHTEMITTIERENDE VORRICHTUNG
DISPOSITIF ÉLECTROLUMINESCENT

(30) Priority: 12.10.2021 EP 21202180
(43) Date of publication of application: 21.08.2024
(73) Proprietor: Signify Holding B.V., 5656 AE Eindhoven (NL)
(72) Inventor: CORNELISSEN, Hugo, Johan, 5656 AE Eindhoven (NL); DE VRIES, Hendrikus, Johan, Adrie, 5656 AE Eindhoven (NL)
(74) Representative: Verweij, Petronella Daniëlle
(86) International application number: PCT/EP2022/078077
(87) International publication number: WO 2023/061926

(56) References cited:
- EP-A1- 2 527 892
- EP-A2- 0 959 295
- EP-A2- 1 094 272
- WO-A1-2017/192795
- WO-A2-98/55798
- AT-B- 374 258
- DE-U1- 20 001 567
- US-A1- 2006 203 493

## Description

### FIELD OF THE INVENTION

The invention relates to a light emitting device comprising a light source adapted for, in operation, emitting light source light, a cavity being diffusely reflective and comprising a light exit plane, the light source being arranged in the cavity, and at least two lamellae arranged extending from a position level with the light exit plane and away from the cavity, the at least two lamellae being spaced apart by a spacing.

### BACKGROUND OF THE INVENTION

It is more and more generally understood that the spread of viruses, such as the COVID-19-virus, can be effectively contained by air disinfection since that destroys the virus in aerosols.

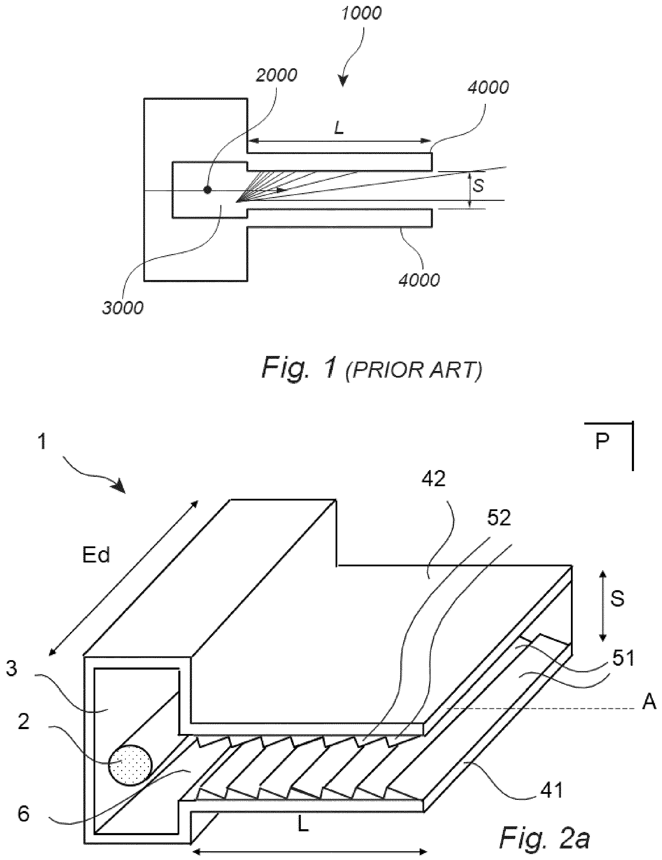

Upper-air disinfection units based on tubular 254 nm sources and lamella optics to collimate the radiation in a narrow beam are known. Such a light emitting device 1000 is shown in a schematic cross-section in Fig. 1. The light emitting device 1000 comprises a light source 2000 adapted for, in operation, emitting light source light, a cavity 3000 being diffusely reflective, and two lamellae 4000 arranged extending from the cavity 3000. The lamellae 4000 are absorbing lamellae.

For instance, EP 0959295 describes a luminaire comprising various flat, lightscattering lamellae extending between elongated reflectors having a longitudinal edge in a luminous window. The lamellae have a concave outer edge in the luminous window and a convex inner edge. Undesirable reflections are precluded by the convex inner edge, while screening of the electric lamp to be accommodated is maintained. The lamellae may comprise folds provided in such a way that the lamellae are undulated in shape. The amplitude of the folds may decrease towards the outer edge.

However, absorption in these lamella causes a significant power loss. Therefore, there is a need for efficiency improvement of these systems.

US 2006/203493 A1 discloses a light fixture for illuminating a specific target area.

### SUMMARY OF THE INVENTION

It is an object of the present invention to overcome this problem, and to provide a light emitting device of the type mentioned by way of introduction which has low or insignificant power losses.

According to a first aspect of the invention, this and other objects are achieved by means of a light emitting device comprising at least one elongated light source extending in an elongation direction Ed and adapted for, in operation, emitting light source light, a cavity being diffusely reflective and comprising a light exit plane, the at least one light source being arranged in the cavity, and at least two lamellae arranged extending from a position level with the light exit plane and away from the cavity, the at least two lamellae being spaced apart by a spacing, where each of the at least two lamellae comprises a first major surface being specular reflective and extending in a direction being parallel with a center axis of a main issue direction of light by the light emitting device and facing the spacing, and where each of the at least two lamellae comprises a first plurality of prismatic elements arranged on the first major surface such that at the first major surface light is reflected by the first plurality of prismatic elements, wherein each prismatic element is elongated along the elongation direction Ed.

Along in the context of the invention is to be understood as at an angle of 0° ± 10°, such as ± 5° or ± 1° or parallel.

By providing that each of the at least two lamellae comprises a first plurality of prismatic elements arranged on the first major surface such that the first major surface of each of the at least two lamellae is configured to be prismatic reflecting, a light emitting device with a collimator that reflects instead of absorbs light propagating in the unwanted directions is provided for.

Thereby, a light emitting device of the type mentioned by way of introduction which has low or insignificant power losses, and which in turn comprises an increased efficiency, has been provided for. The lighting device according to invention is capable and configured to provide, during operation, in a relatively efficient manner a light beam which is relatively narrow beam in a direction perpendicular to the elongation direction and relatively wide in the elongation direction, rendering the lighting device especially suitable for disinfection systems mounted at ceilings of a room and to provide UV light grazing along the ceiling (also known as an upper air disinfection systems).

Furthermore, the above advantages may be obtained without the need for a separate reflector at the light exit window such as it is common in the prior art. Thereby, a light emitting device with a simpler structure and which is more cost efficient to produce is provided for.

In an embodiment, each of the at least two lamellae comprises a second major surface being specular reflective and extending in a direction being parallel with the first major surface and facing away from the spacing, and at least one of the at least two lamellae comprises a second plurality of prismatic elements arranged on the second major surface such that at the second major surface light is reflected by the second plurality of prismatic elements.

Such a light emitting device is especially advantageous when the light emitting device also comprises a plurality of lamellae or an array of lamellae as well as more than one light source and optionally more than one cavity.

Thereby both mutually opposite surfaces the of each of the at least two lamellae is configured to be prismatic reflecting, which provides for a light emitting device with a collimator that reflects instead of absorbs light propagating in the unwanted directions, also in case the light emitting device comprises more than two lamellae, more than one light source and optionally more than one cavity. Thereby, a light emitting device of the type mentioned by way of introduction which has low or insignificant power losses, and which in turn comprises an increased efficiency, has been provided for.

In an embodiment, for each of the at least two lamellae, each of the prismatic elements of the first plurality of prismatic elements and the second plurality of prismatic elements comprise a first facet facing towards the spacing and being adapted for collimating at least a part of light incident thereon and a second facet facing towards the spacing and being adapted for reflecting light incident thereon towards the cavity.

By providing such first facets on each prismatic element, a light emitting device with a highly collimated light output is provided for without needing to add a separate collimating optical element. Thereby, a light emitting device with a simpler structure and which is more cost efficient to produce is provided for.

By providing such second facets, recycling of light is provided by the prismatic elements. More particularly, the radiation density inside the cavity increases due to the back reflection of light at the second facet of each prismatic element. **In** addition to increasing the degree of collimation of the light output, this has the further advantage of being useable to more effectively disinfect air that is forced through the cavity.

In an embodiment, the first facet comprises a first slope and the second facet comprises a second slope, where the first slope comprises a first slope angle with respect to the first major surface and the second slope comprises a second slope angle with respect to the first major surface, where the first slope angle is in the interval of 5° to 35°, and where the second slope angle is in the interval of 90° to 55°.

Combinations of first and second slope angles lying within such intervals have been proven by way of simulations to provide both for a light emitting device with particularly low or insignificant power losses and thus a particularly high efficiency gain, and for a light emitting device producing a particularly well collimated light output.

In an embodiment, the sum of the first slope angle and the second slope angle is between 80° and 110°.

In an embodiment, the sum of the first slope angle and the second slope angle is 90°.

In an embodiment, the first facet and the second facet extend in an angle of between 70° and 100° with respect to one another.

In an embodiment, the first facet and the second facet extend in in an angle of 90° with respect to one another.

Combinations of first and second slope angles with a sum lying within such intervals, and particularly being 90°, as well as combinations of first and second slope angles leading to the first and second facets extending in such angles with respect to one another, have been proven by way of simulations to provide a particularly good trade-off between particularly low or insignificant power losses and thus a particularly high efficiency gain, and a particularly well collimated light output.

In an embodiment, the first slope angle is 5° and the second slope angle is 85°. In an embodiment, the first slope angle is 10° and the second slope angle is 80°. In an embodiment, the first slope angle is 15° and the second slope angle is 75°. In an embodiment, the first slope angle is 20° and the second slope angle is 70°. In an embodiment, the first slope angle is 25° and the second slope angle is 65°. In an embodiment, the first slope angle is 30° and the second slope angle is 60°. In an embodiment, the first slope angle is 35° and the second slope angle is 55°. In an embodiment, the first slope angle is 20° and the second slope angle is 60°. In an embodiment, the first slope angle is 20° and the second slope angle is 80°.

In an embodiment the first slope angle is 20° and the second slope angle is 90°.

The above combinations of first and second slope angles have been proved by way of simulations to provide for a particularly high efficiency gain.

Especially, a surprising finding is that the combination of a first slope angle of 20° and a second slope angle of 70° results in the same FWHM beam shape as the absorbing lamellas used in the prior art light emitting devices, such as that shown in Fig. 1. This combination of first and second slope angle is therefore especially advantageous.

In an embodiment, each of the prismatic elements of at least one of the first plurality of prismatic elements and the second plurality of prismatic elements comprise one or more of a width being less than or equal to 1 mm and a height being less than or equal to 0.3 mm. The prismatic elements typically have a length Pl of at least ten times the width of the prismatic element, such as, for example, 10 mm <= Pl <= 1200 mm, such as Pl = 30 mm, Pl= 50 mm or Pl = 600 mm.

Thereby, a light emitting device is provided for which the prismatic elements are provided with a size that ensures sufficient light manipulation at the prismatic elements to provide the above advantages.

In an embodiment, each of the at least two lamellae comprises a length being at least 10 mm.

Thereby, a light emitting device is provided for which the lamellae are provided with a length ensuring sufficient light manipulation at the prismatic elements while at the same time being kept relatively small such that the light emitting device may be kept compact.

In an embodiment, a width of the spacing between the at least two lamellae is at least 2 mm, the width of the spacing being defined as the shortest distance between the opposite first major surfaces of the respective lamellae.

Thereby, a light emitting device is provided for which the lamellae are provided with a spacing of a size ensuring sufficiently low light losses while at the same time being kept relatively small such that the light emitting device may be kept compact.

In an embodiment, at least one of the cavity, the first plurality of prismatic elements and the second plurality of prismatic elements comprise a reflectivity of at least 90 %. Especially, the first plurality of prismatic elements and the second plurality of prismatic elements comprise a reflectivity of at least 90 % for light with a wavelength in the range of 280 nm to 400 nm.

Thereby, the light losses in the cavity and in the spacing, and especially at the prismatic elements, is lowered even further.

In an embodiment, each of the prismatic elements of the plurality of prismatic elements comprises a height extending in a direction perpendicular to the first major surface, and at least one of the first slope angle, the second slope angle and the height of a given one of the prismatic elements varies as a function of the position of that prismatic element.

Thereby, the light losses at the prismatic elements may be lowered even further, and the collimating effect may be improved.

In an embodiment, at least one of the first slope angle and the second slope angle increases with the distance from the cavity. Especially, a second slope angle which increases with the distance from the cavity may be preferable.

Thereby, the light losses at the prismatic elements may be lowered even further, and the collimating effect may be improved even further.

In an embodiment, the light source is a tubular light source.

In an embodiment, at least one of the surface of the prismatic elements and the surface of the cavity may be made of or may comprise a layer or coating of a diffuse material such as PFTE or a specular material such as polished and fresh Aluminum (Al).

Generally, reflective, or non-absorbing, materials are advantageous, especially in combination with light sources emitting UV-light, as a particularly low loss of light is thereby obtained.

Diffuse materials like PFTE have the advantage of making it easier to maintain over time a high reflectivity.

Specular materials such as Aluminum have the advantage of having a very high reflectivity.

In an embodiment, the light emitting device comprises a plurality of lamellae or an array of lamellae.

Such a light emitting device is especially advantageous when the light emitting device also comprises more than one light source and optionally more than one cavity. In such a case, the plurality of lamellae may ensure sufficient reflection and collimation of light from more than one light source arranged in the same cavity or in separate cavities with particularly low light losses.

Such a light emitting device, which comprises a plurality of lamellae or an array of lamellae, may for instance comprise:
at least two light sources adapted for, in operation, emitting light source light,
a first cavity being diffusely reflective and comprising a first light exit plane, one of the at least two light sources being arranged in the cavity,
a second cavity being diffusely reflective and comprising a second light exit plane, one of the at least two light sources being arranged in the cavity and
at least three lamellae arranged extending from a position level with at least one of the first light exit plane and the second the light exit plane and away from the first cavity and the second cavity,
a first lamella and a second lamella being spaced apart by a first spacing, and the second lamella and a third lamella being spaced apart by a second spacing, wherein
each of the first lamella and the second lamella comprises a first major surface being specular reflective and extending in a direction being parallel with a longitudinal center axis of the light emitting device and facing the first spacing, and each of the first lamella and the second lamella comprises a first plurality of prismatic elements arranged on the first major surface such that at the first major surface light is reflected by the first plurality of prismatic elements, and wherein
each of the second lamella and the third lamella comprises a second major surface being specular reflective and extending in a direction being parallel with a longitudinal center axis of the light emitting device and facing the second spacing, and each of the second lamella and the third lamella comprises a second plurality of prismatic elements arranged on the second major surface such that at the second major surface light is reflected by the second plurality of prismatic elements.

In further embodiments, the first cavity and the second cavity may be one and the same cavity, and/or at least one of the at least three lamellae may extend from the bottom of the cavity.

The light emitting device may have the feature that the light source is configured to emit UV-light, during operation. In particular the light emitting device has an emissions spectrum with a spectral distribution with a highest emission peak in the wavelength range of 100 nm - 400 nm.

The invention further relates to a luminaire or a lamp comprising a light emitting device according to any one of the above claims.
It is noted that the invention relates to all possible combinations of features recited in the claims.

### BRIEF DESCRIPTION OF THE DRAWINGS

This and other aspects of the present invention will now be described in more detail, with reference to the appended drawings showing embodiment(s) of the invention.
Fig. 1 shows a cross-sectional view of a prior art light emitting device.
Figs 2a-b show a cross-sectional views of a light emitting device according to a first embodiment of the invention and comprising two lamellae, each with a plurality of prismatic elements.
Fig. 3 shows an enlarged cross-sectional view of the detail III on Fig. 2b.
Fig. 4 shows an enlarged cross-sectional view of an alternative embodiment of a detail similar to that of Fig. 3.
Fig. 5 shows a cross-sectional view of a light emitting device according to a second embodiment of the invention and comprising three lamellae, each with a plurality of prismatic elements.
Fig. 6 shows a cross-sectional view of a light emitting device according to a third embodiment of the invention and comprising two lamellae, each with a plurality of prismatic elements.
Fig. 7 shows a perspective view of a prismatic ridge/groove of the light emitting device according to Fig. 6.
Fig. 8 shows a plot illustrating the performance of a light emitting device according to Fig. 6.
Fig. 9 shows a plot illustrating the performance of a prior art light emitting device according to Fig. 1.
Fig. 10 shows a plot illustrating the irradiance on a first major surface of a cavity of a light emitting device according to Fig. 6.
Fig. 11 shows a plot illustrating the irradiance on a first major surface of a cavity of a light emitting device according to Fig. 1.
Fig. 12 shows a graph illustrating the resulting power as a function of the lamella spacing for various embodiments of lamellae, and thus the efficiency of the various embodiments of lamellae, of a light emitting device according to Fig. 6.
Fig. 13 shows a graph illustrating the resulting Full Width at Half Maximum (FWHM) as a function of the lamella spacing for the same embodiments of lamellae of a light emitting device according to Fig. 6 as shown in Fig. 12.

As illustrated in the figures, the sizes of layers and regions are exaggerated for illustrative purposes and, thus, are provided to illustrate the general structures of embodiments of the present invention. Like reference numerals refer to like elements throughout.

### DETAILED DESCRIPTION

The present invention will now be described more fully hereinafter with reference to the accompanying drawings, in which currently preferred embodiments of the invention are shown. This invention may, however, be embodied in many different forms and should not be construed as limited to the embodiments set forth herein; rather, these embodiments are provided for thoroughness and completeness, and fully convey the scope of the invention to the skilled person.

Referring first to Figs. 2a-b, cross sectional side views of a light emitting device 1 according to the invention is shown. The light emitting device 1 generally, and irrespective of the embodiment, comprises an elongated light source 2 elongated in an elongation direction Ed and adapted for, in operation, emitting light source light, a cavity 3 being diffusely reflective and comprising a light exit plane 6, and at least two lamellae 41, 42 arranged extending from the light exit plane 6 of the cavity 3. The light emitting device 1 further comprises a longitudinal center axis A.

The light source 2 is arranged in the cavity 3. The light source 2 may in principle be any type of light source, such as an array/linear row of LEDs, an excimer light source, an LED retrofit TL, or a laser diode light source. For instance, the light source 2 may be a tubular light source. The light source 2 may be arranged on a substrate which may comprise wiring for electrical supply of the light source 2. The substrate may be a PCB. The light source 2 may be configured to emit visible light, UV light or a combination thereof. The spectral distribution of the UV light may comprise any one or more of one or more wavelengths within the UV-C spectrum, 100 nm - 280 nm, one or more wavelengths within the UV-A spectrum, 320 nm - 400 nm, and one or more wavelengths with the UV-B spectrum, 280 nm - 320 nm. It is also feasible to provide the light emitting device according to the invention with two (or more) light sources 2, of which one may be configured to emit visible light, and another may be configured to emit UV light.

The cavity 3 is diffusely reflective. The cavity 3 comprises a circumferential first major surface 31 and an opening extending in the light exit plane 6. The circumferential surface 31 comprises a surface part forming a bottom surface 32 extending opposite to, and optionally also parallel with, the light exit plane 6. Light emitted by the light source 2 is reflected off the circumferential surface 31 of the cavity 3 and eventually leaves the cavity 3 through the opening or at the light exit plane 6. The cavity 3 may comprise a reflectivity of at least 90 %.

The lamellae 41 and 42 are arranged extending away from the cavity 3. The lamellae 41 and 42 are arranged at and attached to a wall or surface 7, in which the cavity 3 is provided, on opposite sides of the cavity 3. The lamella 41 comprises a first major surface 411, a second major surface 412 and an end surface 413. The first major surface 411 is specular reflective. The second major surface 412 may optionally be specular reflecting. The end surface 413 extends between the first major surface 411 and the second major surface 412 opposite to the cavity 3. The first major surface 411 and the second major surface 412 extend parallel to one another. The lamella 42 comprises a first major surface 421, a second major surface 422 and an end surface 423. The first major surface 421 is specular reflecting. The second major surface 422 may optionally be specular reflective. The end surface 423 extends between the first major surface 421 and the second major surface 422 opposite to the cavity 3. The first major surface 421 and the second major surface 422 extend parallel to one another. The lamellae 41 and 42 are separated by a spacing 9. The spacing 9 comprises a width S. The width S is defined as the shortest distance between the opposite first major surfaces 411 and 421 of the respective lamellae 41 and 42. The width S is equal to or smaller than a width 8 of the opening of the cavity 3. Each of the at least two lamellae 41 and 42 further comprises a length L measured perpendicular to the width S of the spacing 9. The length L is chosen to be at least 10 mm. The width S of the spacing 9 between the at least two lamellae 41, 42 is chosen to be at least 2 mm.

The lamellae 41 and 42 are configured to be prismatic reflecting. More particularly, at least one of the first major surface 411, 421 and the second major surface 412, 422 of each of the lamellae 41 and 42 are configured to be prismatic reflecting. Each of the lamellae 41 and 42 thus comprises a plurality of prismatic elements 51 and 52, respectively. The plurality of prismatic elements 51 and 52 are in the embodiment shown in Fig. 2a-b arranged on the respective first major surfaces 411 and 421 of the respective lamella 41 and 42. Alternatively, or additionally, a further plurality of prismatic elements may be arranged on the respective second major surfaces 412 and 422 of the respective lamella 41 and 42.

In Fig. 2a the cross sectional side view is shown in perspective to illustrate that the prismatic elements 51, 52 are prismatic ridges or prismatic grooves elongated along the elongation direction Ed of the light source 2. Said elongation direction Ed is normal to a plane P in which the width S and the length L are measured.

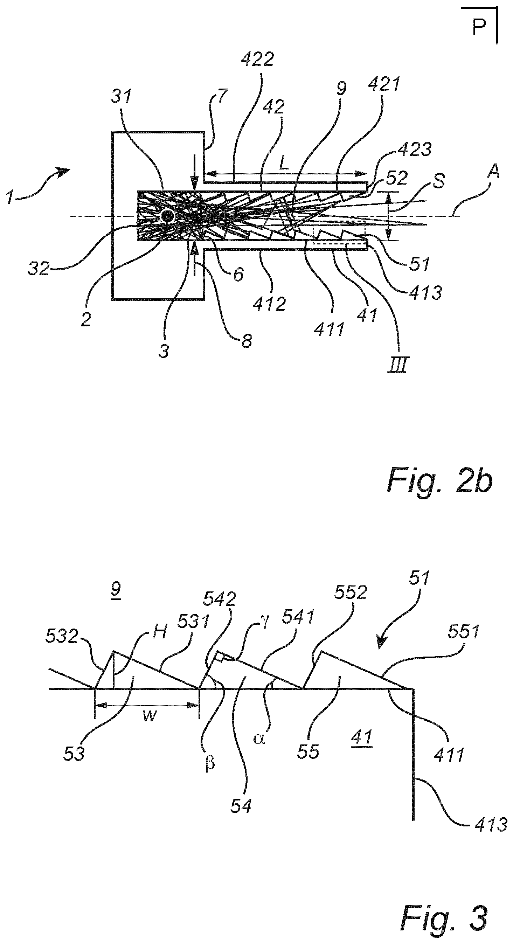

Referring now also to Fig. 3, an enlarged cross-sectional view of a detail of the lamella 41 is shown. It is noted that the following description of the prismatic elements of the plurality of prismatic elements 51 also applies to the prismatic elements of the plurality of prismatic elements 52 of the lamella 42.

Each of the prismatic elements 53, 54, 55 of the plurality of prismatic elements 51 are in this embodiment triangular prismatic elements. Each of the prismatic elements 53, 54, 55 of the plurality of prismatic elements 51 comprise a first facet 531, 541, 551 and a second facet 532, 542, 552. Each first facet 531, 541, 551 and each second facet 532, 542, 552 face towards the spacing 9 between the lamellae 41, 42. Each first facet 531, 541, 551 is adapted for collimating at least a part of light incident thereon. Each second facet 532, 542, 552 is adapted for reflecting light incident thereon towards the cavity 3. Each first facet 531, 541, 551 comprises a first slope and each second facet 532, 542, 552 comprises a second slope. Each first facet 531, 541, 551 slopes towards the end surface 413 of the lamella 41, and each second facet 532, 542, 552 slopes away from the end surface 413 of the lamella 41. In other words, each first facet 531, 541, 551 slopes away from the cavity 3, and each second facet 532, 542, 552 slopes towards the cavity 3. The first slope comprises a first slope angle α with respect to the first major surface. The second slope comprises a second slope angle β with respect to the first major surface. The first slope angle α is chosen to lie in the interval of 5° to 35°. The second slope angle β is chosen to lie in interval of 90° to 55°. The sum of the first slope angle α and the second slope angle β, which corresponds to the top angle γ shown on Fig. 3, is thus in the embodiment shown 90°.

Each of the prismatic elements 53, 54, 55 of the plurality of prismatic elements 51 further comprises a height H and a width W. The height H is measured in a direction perpendicular to the first major surface 411 of the lamella 41 on which the prismatic groove (or ridge) 51 is arranged as the distance between the top angle of a prismatic ridge (or groove) and the first major surface 411. The width W is measured perpendicular to the height H. The width W of each of the prismatic elements 53, 54, 55 of the plurality of prismatic elements 51 is chosen to be less than or equal to 1 mm. The height H of each of the prismatic elements 53, 54, 55 of the plurality of prismatic elements 51 is chosen to be less than or equal to 0.3 mm. The plurality of prismatic elements 51 may further comprise a reflectivity of at least 90 %.

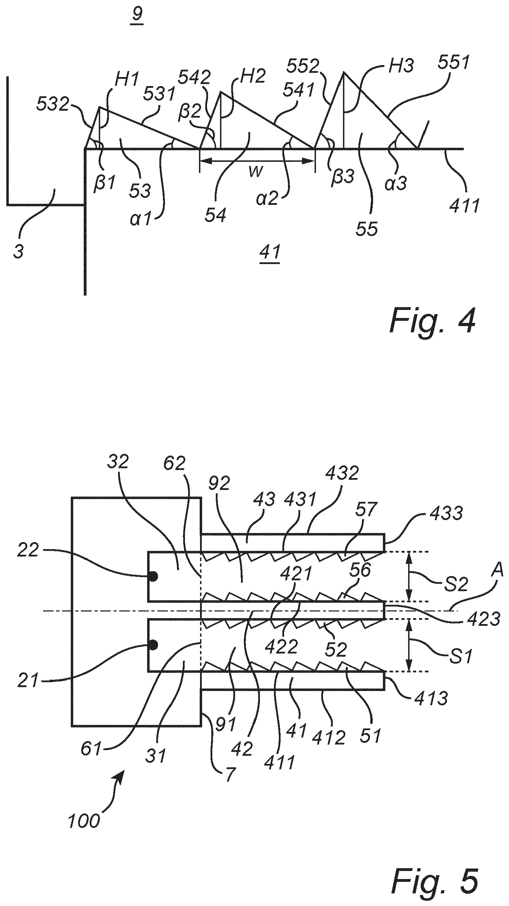

Referring now to Fig. 4, an enlarged cross-sectional view of a detail of the lamella 41 is shown. It is noted that the following description of the prismatic elements of the plurality of prismatic elements 51 also applies to the prismatic elements of the plurality of prismatic elements 52 of the lamella 42. The lamella 41 shown in Fig. 4 differs from that of Fig. 3 in virtue of the prismatic elements having a different configuration.

More particularly, the second slope angle β of the prismatic elements 53, 54, 55 of the lamella 41 shown in Fig. 4 varies as a function of the position of that prismatic element such that the second slope angle β increases with the distance from the cavity 3. In other words, the second slope angle β3 of the prismatic element 55, which is the farthest from the cavity 3, is larger than the second slope angle β2 of the prismatic element 54, which in turn is larger than the second slope angle β1 of the prismatic element 53, which is the closest to the cavity 3.

Alternatively, or additionally, the first slope angle α of the prismatic elements 53, 54, 55 of the lamella 41 may vary as a function of the position of that prismatic element such that the first slope angle α increases (or decreases) with the distance from the cavity 3. In other words, the first slope angle α3 of the prismatic element 55, which is the farthest from the cavity 3, may be larger (or smaller) than the first slope angle α2 of the prismatic element 54, which in turn may be larger (or smaller) than the first slope angle α1 of the prismatic element 53, which is the closest to the cavity 3.

Alternatively, or additionally, the height H of the prismatic elements 53, 54, 55 of the lamella 41 may vary as a function of the position of that prismatic element such that the height H increases (or decreases) with the distance from the cavity 3. In other words, the height H3 of the prismatic element 55, which is the farthest from the cavity 3, may be larger (or smaller) than the height H2 of the prismatic element 54, which in turn may be larger (or smaller) than the height H1 of the prismatic element 53, which is the closest to the cavity 3.

Referring now to Fig. 5, a cross sectional side view of an alternate embodiment of a light emitting device 100 according to the invention is shown. The light emitting device 100 differs from that of Fig. 2a-b described above in virtue of the following features.

The light emitting device 100 comprises two cavities 31 and 32 each comprising an opening extending in a light exit plane 61 and 62, respectively. A light source 21 and 22, respectively, is arranged in each cavity 31 and 32. The light emitting device 100 further comprises a plurality of lamellae, and more particularly three lamellae 41, 42 and 43.

Each of the three lamellae 41, 42, 43 comprises a first major surface 411, 421 and 431, respectively, a second major surface 412, 422 and 432, respectively, and an end surface 413, 423 and 433, respectively. The lamella 41 comprises a plurality of prismatic elements 51 provided on the first major surface 411. The lamella 42 comprises a plurality of prismatic elements 52 provided on the first major surface 421 and a plurality of prismatic elements 53 provided on the second major surface 422. The lamella 43 comprises a plurality of prismatic elements 54 provided on the first major surface 431. Each of the pluralities of prismatic elements 51-54 may be constructed with facets and slopes as described above in connection with Figs. 2-4. It is thus generally feasible, that a lamella of a light emitting device according to the invention may be provided with prismatic elements on both its first major surface and its second major surface.

The lamellae 41 and 42 are arranged extending away from the cavity 31. The lamellae 41 and 42 are arranged at and attached to a wall or surface 7, in which the cavity 31 is provided, on opposite sides of the cavity 31. The lamellae 41 and 42 are spaced apart with a spacing 91 having a width S1. The lamellae 42 and 43 are arranged extending away from the cavity 32. The lamellae 41 and 42 are arranged at and attached to a wall or surface 7, in which the cavity 32 is provided, on opposite sides of the cavity 32. The lamellae 42 and 43 are spaced apart with a spacing 92 having a width S2. Thus, the lamella 42 is arranged extending from the wall or surface 7 between the cavities 31 and 32.

In this way, the pluralities of prismatic elements 51 and 52 are arranged and adapted for collimating at least a part of light emitted by the light source 21 and propagating in the spacing 91 and for reflecting at least a part of the light emitted by the light source 21 and propagating in the spacing 91 towards the cavity 31. Similarly, the pluralities of prismatic elements 53 and 54 are arranged and adapted for collimating at least a part of light emitted by the light source 22 and propagating in the spacing 92 and for reflecting at least a part of the light emitted by the light source 22 and propagating in the spacing 92 towards the cavity 32.

It is also feasible that the plurality of lamellae may comprise further lamellae of the same type and construction of the lamellae 41 and 42. The said further lamellae are arranged extending away from the cavity 31 and are rotated 90 degrees with respect to the lamellae 41 and 42, such that the said further lamellae are arranged at and attached to a wall or surface 7, in which the cavity 31 and 32 is provided, on opposite sides of the cavity 31 or 32 and with the same spacing S1 or S2 as the lamellae 41. Thereby, the light emitting device is provided with a grid of lamellae. Thereby the prismatic elements of the said further lamellae face the spacing 91 or 92 on the two sides not covered by the lamellae 41 and 42. Providing such further lamellae will achieve collimation in two directions, instead of only in one direction.

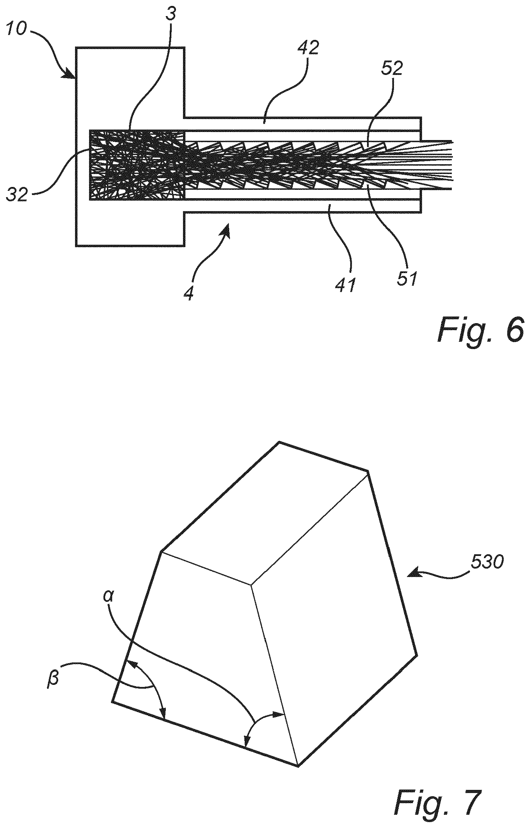

Referring now to Figs. 6, a cross sectional side view of an alternate embodiment of a light emitting device 10 according to the invention is shown. Referring also to Fig. 7 an enlarged view of a prismatic element 530 of the pluralities of prismatic elements 51 and 52 of the light emitting device 10 is shown. The light emitting device 10 differs from those of Figs. 2 and 5 described above in virtue of the construction of the prismatic elements 530 as shown in Fig. 7.

The prismatic element 530 is a trapezoidal prismatic element, but otherwise comprises facets and slopes with slope angles as described above in connection with the light emitting device 1 shown in Fig. 2a-b. Other types and shapes of prismatic elements are also feasible.

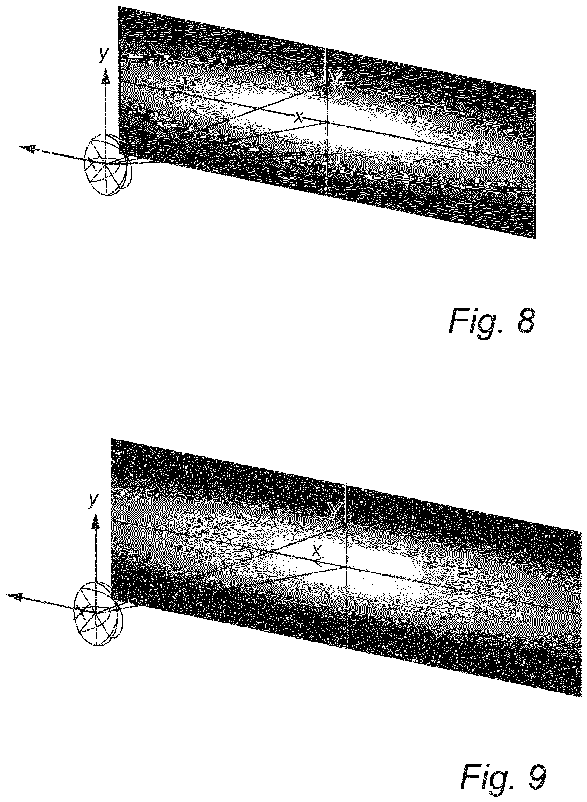

In the following the performance of a light emitting device according to the invention will be described by way of an example in which measurements were performed on a light emitting device 10 of the type shown in Fig. 6 and with prismatic elements 530 of the type shown in Fig. 7. More particularly, the prismatic elements 530 were of the type shown in Fig. 7 and with a first slope angle of 20°, a second slope angle of 70° and a width W of 1 mm. The lamellae 41 and 42 of the light emitting device 10 were provided with a length L of 10 mm and arranged with a spacing 9 having a width S of 1.5 mm. Both the lamellae 41 and 42 and the cavity 3 were provided with a reflectivity of 90 %. The performance of the light emitting device 10 was evaluated using the illumination design software tool LightTools. The results are shown in Figs. 8, 10, 12 and 13.

Fig. 8 shows a plot illustrating the intensity of the light emitted by the light emitting device 10 on a screen with a size of 4x1 m at a distance of 2 m from the light emitting device. The resulting Full Width at Half Maximum (FWHM) is 10.6 degrees. For comparison, Fig. 9 shows a similar plot illustrating the intensity of the light emitted by a prior art light emitting device 1000 with absorbing lamellae 4000 according to Fig. 1. The resulting FWHM is 11.5 degrees. As may be seen, with a light emitting device 10 according to the invention a considerably smaller FWHM, and thus a considerably more collimated output light beam, is obtained as compared to the prior art light emitting devices.

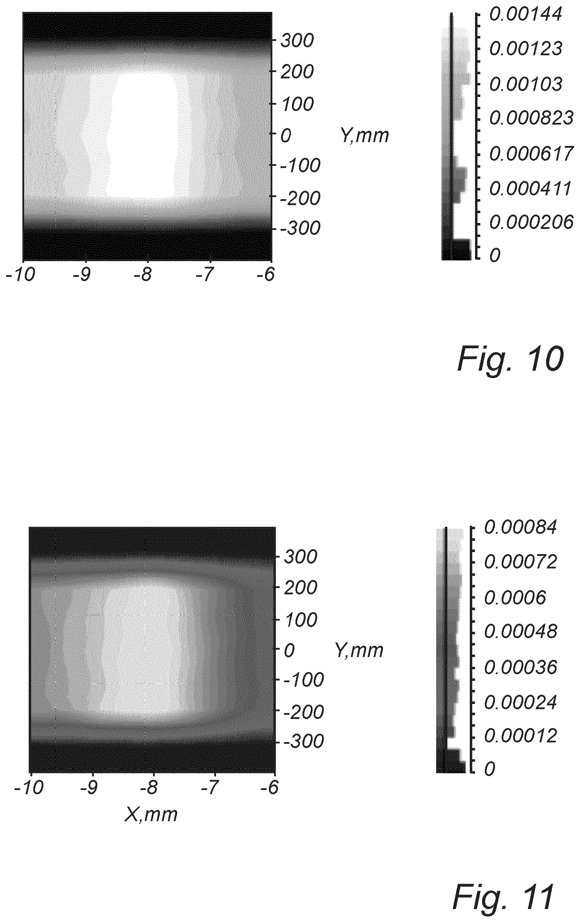

Fig. 10 shows a plot illustrating the irradiance in W/mm² as a function of position on a bottom surface of the cavity 3 of the light emitting device 10. For comparison, Fig. 11 shows a plot illustrating the irradiance in W/mm² as a function of position on a bottom surface of a cavity 3000 of a prior art light emitting device 1000 according to Fig. 1. As may be seen the irradiance on the bottom surface of the cavity 3 of the light emitting device 10 is considerably higher over a considerably larger area of the surface as compared to the prior art light emitting device 1000. This illustrates the effect of the facets configured to reflect light back into the cavity of the prismatic elements of a light emitting device according to the invention. Particularly, the average irradiance on the bottom surface of the cavity is seen to increase by about a factor of two as compared to the prior art light emitting devices due to recycling of light provided by the prismatic elements. With a light emitting device according to the present invention, the radiation density inside the cavity 3 thus increases due to the back reflection of light at the prismatic elements. This can be used to more effectively disinfect air that is forced through the cavity 3.

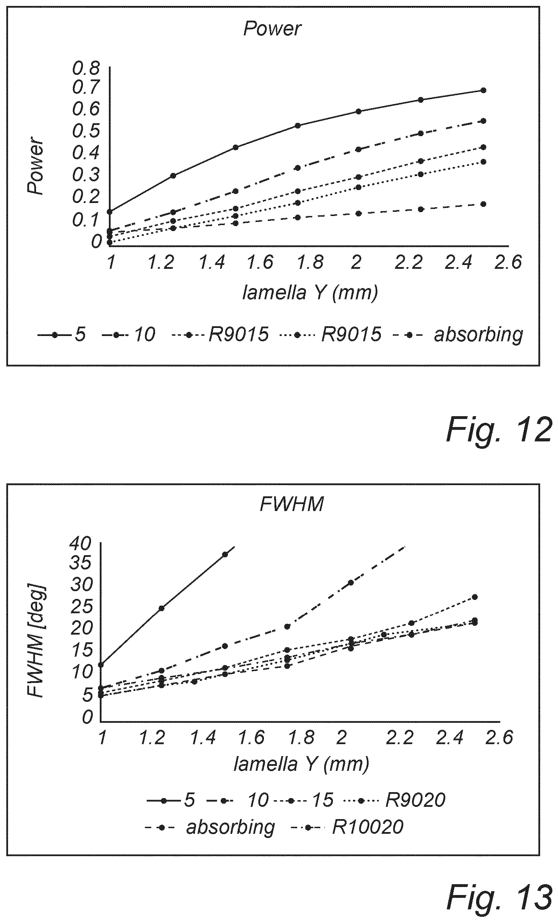

Fig. 12 shows a graph illustrating the resulting power as a function of the width S of the lamella spacing 9 for various embodiments of lamellae, and thus the efficiency of the various embodiments of lamellae, of a light emitting device according to Fig. 6 and comprising a tubular light source 2. Four slope angle combinations are shown, namely seen from the top and downwards: 5-85, 10-70, 15-75 and 20-70 degrees, respectively. In all cases the reflectivity of the cavity 3 was 90 %, and the reflectivity of the prismatic elements were 90 %. For comparison, the dashed line illustrates the power resulting from a prior art light emitting device 1000.

Fig. 13 shows a graph illustrating the resulting Full Width at Half Maximum (FWHM) as a function of the width S of the lamella spacing 9 for the same embodiments of lamellae of a light emitting device 10 according to Fig. 6 as shown in Fig. 12. Thus, again four slope angle combinations are shown, namely seen from the top and downwards: 5-85, 10-70, 15-75 and 20-70 degrees, respectively. Furthermore, two versions of the slope angle combination 20-70 degrees are shown, namely with a reflectivity of the cavity being 90 % and 100 %, respectively - cf. the two lowermost full lines being almost coinciding. For comparison, the dashed line illustrates the FWHM resulting from a prior art light emitting device 1000.

As may be seen from Fig. 12, the efficiency gain depends on the beam angle compared to absorbing lamellas. For a slope angle combination of 20-70 degrees, the efficiency gain equals 1.3 to 2.0 times that of the prior art light emitting device 1000. As may be seen from Fig. 13, the FWHM is determined by the lamella length L and lamella spacing width S. In the example illustrated the FWHM lies in the interval from 5° to >30° for lamella coordinates Y of ±1 mm to ±2.5 mm. The lamella spacing width S is given by 2*(Y - 0.5) mm. For a slope angle combination of 15-75 degrees a slightly larger FWHM results (Fig. 13), but the efficiency gain is higher as well at 1.7 - 2.3 times that of the prior art light emitting device 1000 (Fig. 12). As may also be seen from Fig. 13, for a slope angle combination of 20-70 degrees the resulting FWHM is almost identical to the absorbing lamellas, and the FWHM does not depend on the reflectivity of the cavity.

To minimize self-absorption in the (tubular) light source 2, a large cavity and a small source as compared to each other would be preferable. Self-absorption in the (tubular) light source 2 may play a role in the final efficiency gain of the light emitting device according to the invention.

The prismatic elements 53, 54, 55 and the surface of the cavity 3 may be made of or comprising a layer or coating of a diffuse material such as PFTE or a specular material such as polished and fresh Al. Diffuse materials like PFTE have the advantage of making it easier to maintain over time a high reflectivity.

The light emitting device 1, 10, 100 according to the present invention may form part of a luminaire or a lamp. A lamp may comprise a light emitting device 1, 10, 100 according to the present invention in combination with a base for mounting in a socket and for being electrically connected via the socket to an external power source. A luminaire may comprise a light emitting device 1, 10, 100 according to the present invention and a housing accommodating and holding the light emitting device 1, 10, 100. A luminaire often also comprises electronics and electrical contacting means for driving the light emitting device 1, 10, 100 and electrically connecting the luminaire (and light emitting device 1, 10, 100) with an external or internal power source. Such a luminaire may for instance take the form of a light bulb.

The person skilled in the art realizes that the present invention by no means is limited to the preferred embodiments described above. On the contrary, many modifications and variations are possible within the scope of the appended claims.

Additionally, variations to the disclosed embodiments can be understood and effected by the skilled person in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage.

## Claims

1. A light emitting device (1) comprising:
at least one elongated light source (2) extending in an elongation direction Ed and adapted for, in operation, emitting light source light,
a cavity (3) being diffusely reflective and comprising a light exit plane (6), the at least one light source being arranged in the cavity, and
at least two lamellae (41, 42) arranged extending from a position level with the light exit plane and away from the cavity, the at least two lamellae being spaced apart by a spacing (9), wherein
each of the at least two lamellae (41, 42) comprises a first major surface (411, 421) being specular reflective and extending in a direction being parallel with a center axis (A) of a main issue direction of light by the light emitting device and facing the spacing, and wherein
at least one of the at least two lamellae (41, 42) comprises a first plurality of prismatic elements (51, 52) arranged on the first major surface (411, 421) such that at the first major surface light is reflected by the first plurality of prismatic elements, **characterised in that**
each prismatic element is elongated along the elongation direction Ed

2. A light emitting device according to claim 1, wherein each of the at least two lamellae (41, 42) comprises a second major surface (412, 422) being specular reflective and extending in a direction being parallel with the first major surface and facing away from the spacing, and wherein
at least one of the at least two lamellae (41, 42) comprises a second plurality of prismatic elements (56, 57) arranged on the second major surface (412, 422) such that at the second major surface light is reflected by the second plurality of prismatic elements.

3. A light emitting device according to any one of the above claims, wherein, for each of the at least two lamellae (41, 42), each of the prismatic elements (53, 54, 55) of the first plurality of prismatic elements and the second plurality of prismatic elements comprise a first facet (531, 541, 551) facing towards the spacing and being adapted for collimating at least a part of light incident thereon and a second facet (532, 542, 552) facing towards the spacing and being adapted for reflecting light incident thereon towards the cavity.

4. A light emitting device according to claim 3, wherein the first facet (531, 541, 551) comprises a first slope and the second facet (532, 542, 552) comprises a second slope, wherein the first slope comprises a first slope angle (α) with respect to the first major surface and the second slope comprises a second slope angle (β) with respect to the first major surface, wherein the first slope angle (α) is in the interval of 5° to 35°, and wherein the second slope angle (β) is in the interval of 90° to 55°.

5. A light emitting device according to claim 4, wherein the sum of the first slope angle (α) and the second slope angle (β) is between 80° and 110°, or wherein the sum of the first slope angle (α) and the second slope angle (β) is 90°.

6. A light emitting device according to claim 4 or 5, wherein:
the first slope angle (α) is 5° and the second slope angle (β) is 85°, or
the first slope angle (α) is 10° and the second slope angle (β) is 80°, or
the first slope angle (α) is 15° and the second slope angle (β) is 75°, or
the first slope angle (α) is 20° and the second slope angle (β) is 70°, or
the first slope angle (α) is 25° and the second slope angle (β) is 65°, or
the first slope angle (α) is 30° and the second slope angle (β) is 60°, or
the first slope angle (α) is 35° and the second slope angle (β) is 55°, or
the first slope angle (α) is 20° and the second slope angle (β) is 60°, or
the first slope angle (α) is 20° and the second slope angle (β) is 80°, or
the first slope angle (α) is 20° and the second slope angle (β) is 90°.

7. A light emitting device according to any one of claims 3 to 6, wherein the first facet and the second facet extend in an angle (γ) of between 70° and 100° with respect to one another or in an angle (γ) of 90° with respect to one another.

8. A light emitting device according to any one of the above claims, wherein each of the prismatic elements (53, 54, 55) of at least one of the first plurality of prismatic elements and the second plurality of prismatic elements comprise one or more of a width (W) being less than or equal to 1 mm and a height (H) being less than or equal to 0.3 mm.

9. A light emitting device according to any one of the above claims, wherein each of the at least two lamellae (41, 42) comprises a length (L) being at least 10 mm.

10. A light emitting device according to any one of the above claims, wherein a width (S) of the spacing (9) between the at least two lamellae is at least 2 mm, the width (S) of the spacing being defined as the shortest distance between the opposite first major surfaces (411, 421) of the respective lamellae (41, 42).

11. A light emitting device according to any one of the above claims, wherein at least one of the cavity (3), the first plurality of prismatic elements (51) and the second plurality of prismatic elements (52) comprise a reflectivity of at least 90 % for light with a wavelength in the range of 280 nm to 400 nm.

12. A light emitting device according to any one of the above claims 4-10, wherein each of the prismatic elements (53, 54, 55) of the first plurality of prismatic elements (51) and the second plurality of prismatic elements (52) comprises a height (H) extending in a direction perpendicular to the first major surface (411), and wherein at least one of the first slope angle (α), the second slope angle (β) and the height (H) of a given one of the prismatic elements varies as a function of the position of that prismatic element.

13. A light emitting device according to claim 12, wherein at least one of the first slope angle (α) and the second slope angle (β) increases with the distance from the cavity.

14. A light emitting device according to any one of the above claims wherein the light source is configured to emit UV-light, during operation, having a spectral distribution with a highest emission peak in the wavelength range of 100 nm - 400 nm.

15. A luminaire or a lamp comprising a light emitting device according to any one of the above claims.

## Patentansprüche

1. Lichtemittierende Vorrichtung (1), umfassend:
mindestens eine längliche Lichtquelle (2), die sich in einer Längsrichtung Ed erstreckt und angepasst ist, um, in Betrieb, Lichtquellenlicht zu emittieren,
einen Hohlraum (3), der diffus reflektierend ist und umfassend eine Lichtaustrittsebene (6), wobei die mindestens eine Lichtquelle in dem Hohlraum angeordnet ist, und
mindestens zwei Lamellen (41, 42), die angeordnet sind, um sich von einer Position auf Höhe der Lichtaustrittsebene und weg von dem Hohlraum zu erstrecken, wobei die mindestens zwei Lamellen durch einen Abstand (9) beabstandet sind, wobei
jede der mindestens zwei Lamellen (41, 42) eine erste Hauptoberfläche (411, 421) umfasst, die spiegelnd reflektierend ist und sich in eine Richtung erstreckt, die parallel zu einer Mittelachse (A) einer Hauptausgaberichtung des Lichts durch die lichtemittierende Vorrichtung ist und dem Abstand zugewandt ist, und wobei
mindestens eine der mindestens zwei Lamellen (41, 42) eine erste Vielzahl von prismatischen Elementen (51, 52) umfasst, die auf der ersten Hauptoberfläche (411, 421) derart angeordnet sind, dass an der ersten Hauptoberfläche Licht durch die erste Vielzahl von prismatischen Elementen reflektiert wird, **dadurch gekennzeichnet, dass**
jedes prismatische Element entlang der Längsrichtung Ed verlängert ist.

2. Lichtemittierende Vorrichtung nach Anspruch 1, wobei jede der mindestens zwei Lamellen (41, 42) eine zweite Hauptoberfläche (412, 422) umfasst, die spiegelnd reflektierend ist und sich in eine Richtung erstreckt, die parallel zu der ersten Hauptoberfläche ist und von dem Abstand abgewandt ist, und wobei
mindestens eine der mindestens zwei Lamellen (41, 42) eine zweite Vielzahl von prismatischen Elementen (56, 57) umfasst, die auf der zweiten Hauptoberfläche (412, 422) derart angeordnet sind, dass an der zweiten Hauptoberfläche Licht durch die zweite Vielzahl von prismatischen Elementen reflektiert wird.

3. Lichtemittierende Vorrichtung nach einem der vorstehenden Ansprüche, wobei für jede der mindestens zwei Lamellen (41, 42) jedes der prismatischen Elemente (53, 54, 55) der ersten Vielzahl von prismatischen Elementen und der zweiten Vielzahl von prismatischen Elementen eine erste Facette (531, 541, 551), die in Richtung des Abstands zeigt und angepasst ist, um mindestens einen Teil des darauf einfallenden Lichts zu kollimieren, und eine zweite Facette (532, 542, 552) umfasst, die in Richtung des Abstands zeigt und angepasst ist, um darauf einfallendes Licht in Richtung des Hohlraums zu reflektieren.

4. Lichtemittierende Vorrichtung nach Anspruch 3, wobei die erste Facette (531, 541, 551) eine erste Neigung umfasst und die zweite Facette (532, 542, 552) eine zweite Neigung umfasst, wobei die erste Neigung einen ersten Neigungswinkel (α) in Bezug auf die erste Hauptoberfläche umfasst und die zweite Neigung einen zweiten Neigungswinkel (β) in Bezug auf die erste Hauptoberfläche umfasst, wobei der erste Neigungswinkel (α) in dem Intervall von 5° bis 35° liegt und wobei der zweite Neigungswinkel (β) in dem Intervall von 90° bis 55° liegt.

5. Lichtemittierende Vorrichtung nach Anspruch 4, wobei die Summe aus dem ersten Neigungswinkel (α) und dem zweiten Neigungswinkel (β) zwischen 80° und 110° beträgt, oder wobei die Summe aus dem ersten Neigungswinkel (α) und dem zweiten Neigungswinkel (β) 90° beträgt.

6. Lichtemittierende Vorrichtung nach Anspruch 4 oder 5, wobei:
der erste Neigungswinkel (α) 5° beträgt und der zweite Neigungswinkel (β) 85° beträgt, oder
der erste Neigungswinkel (α) 10° beträgt und der zweite Neigungswinkel (β) 80° beträgt, oder
der erste Neigungswinkel (α) 15° beträgt und der zweite Neigungswinkel (β) 75° beträgt, oder
der erste Neigungswinkel (α) 20° beträgt und der zweite Neigungswinkel (β) 70° beträgt, oder
der erste Neigungswinkel (α) 25° beträgt und der zweite Neigungswinkel (β) 65° beträgt, oder
der erste Neigungswinkel (α) 30° beträgt und der zweite Neigungswinkel (β) 60° beträgt, oder
der erste Neigungswinkel (α) 35° beträgt und der zweite Neigungswinkel (β) 55° beträgt, oder
der erste Neigungswinkel (α) 20° beträgt und der zweite Neigungswinkel (β) 60° beträgt, oder
der erste Neigungswinkel (α) 20° beträgt und der zweite Neigungswinkel (β) 80° beträgt, oder
der erste Neigungswinkel (α) 20° beträgt und der zweite Neigungswinkel (β) 90° beträgt.

7. Lichtemittierende Vorrichtung nach einem der Ansprüche 3 bis 6, wobei sich die erste Facette und die zweite Facette in einem Winkel (γ) zwischen 70° und 100° zueinander oder in einem Winkel (γ) von 90° zueinander erstrecken.

8. Lichtemittierende Vorrichtung nach einem der vorstehenden Ansprüche, wobei jedes der prismatischen Elemente (53, 54, 55) von mindestens einem der ersten Vielzahl von prismatischen Elementen und der zweiten Vielzahl von prismatischen Elementen eines oder mehrere aufweist von einer Breite (W) von weniger als oder gleich 1 mm und einer Höhe (H) von weniger als oder gleich 0,3 mm.

9. Lichtemittierende Vorrichtung nach einem der vorstehenden Ansprüche, wobei jede der mindestens zwei Lamellen (41, 42) eine Länge (L) von mindestens 10 mm umfasst.

10. Lichtemittierende Vorrichtung nach einem der vorstehenden Ansprüche, wobei eine Breite (S) des Abstands (9) zwischen den mindestens zwei Lamellen mindestens 2 mm beträgt, wobei die Breite (S) des Abstands als die kürzeste Entfernung zwischen den gegenüberliegenden ersten Hauptoberflächen (411, 421) der jeweiligen Lamellen (41, 42) definiert ist.

11. Lichtemittierende Vorrichtung nach einem der vorstehenden Ansprüche, wobei mindestens eines der Hohlräume (3), der ersten Vielzahl von prismatischen Elementen (51) und der zweiten Vielzahl von prismatischen Elementen (52) eine Reflektivität von mindestens 90 % für Licht mit einer Wellenlänge in dem Bereich von 280 nm bis 400 nm umfasst.

12. Lichtemittierende Vorrichtung nach einem der Ansprüche 4 bis 10, wobei jedes der prismatischen Elemente (53, 54, 55) der ersten Vielzahl von prismatischen Elementen (51) und der zweiten Vielzahl von prismatischen Elementen (52) eine Höhe (H) umfasst, die sich in einer Richtung senkrecht zu der ersten Hauptoberfläche (411) erstreckt, und wobei mindestens eines des ersten Neigungswinkels (α), des zweiten Neigungswinkels (β) und der Höhe (H) eines bestimmten einen des prismatischen Elements in Abhängigkeit von der Position dieses prismatischen Elements variiert.

13. Lichtemittierende Vorrichtung nach Anspruch 12, wobei mindestens einer des ersten Neigungswinkels (α) und des zweiten Neigungswinkels (β) mit der Entfernung von dem Hohlraum zunimmt.

14. Lichtemittierende Vorrichtung nach einem der vorstehenden Ansprüche, wobei die Lichtquelle konfiguriert ist, um während des Betriebs UV-Licht, das eine spektrale Verteilung mit einem höchsten Emissionspeak in dem Wellenlängenbereich von 100 nm bis 400 nm aufweist, emittiert.

15. Leuchte oder Lampe, umfassend eine lichtemittierende Vorrichtung nach einem der vorstehenden Ansprüche.

## Revendications

1. Dispositif électroluminescent (1) comprenant :
au moins une source lumineuse allongée (2) s'étendant dans un direction d'allongement Ed et conçue pour émettre, en fonctionnement, une lumière de source lumineuse,
une cavité (3) étant réfléchissante de manière diffuse et comprenant un plan de sortie de lumière (6), l'au moins une source lumineuse étant agencée dans la cavité, et
au moins deux lamelles (41, 42) agencées s'étendant à partir d'une position à niveau avec le plan de sortie la lumière et à l'écart de la cavité, les au moins deux lamelles étant espacées d'un espacement (9), dans lequel
chacune des au moins deux lamelles (41, 42) comprend une première surface principale (411, 421) étant réfléchissante de manière spéculaire et s'étendant dans une direction étant parallèle à un axe central (A) d'une direction principale d'émission de lumière par le dispositif électroluminescent et faisant face à l'espacement, et dans lequel
au moins une des au moins deux lamelles (41, 42) comprend une première série d'éléments prismatiques (51, 52) agencés sur la première surface principale (411, 421) de telle sorte qu'au niveau de la première surface principale la lumière est réfléchie par la première série d'éléments prismatiques, **caractérisé en ce que**
chaque élément prismatique est allongé le long de la direction d'allongement Ed.

2. Dispositif électroluminescent selon la revendication 1, dans lequel chacune des au moins deux lamelles (41, 42) comprend une seconde surface principale (412, 422) étant réfléchissante de manière spéculaire et s'étendant dans une direction étant parallèle à la première surface principale et opposée à l'espacement, et dans lequel
au moins une des au moins deux lamelles (41, 42) comprend une seconde pluralité d'éléments prismatiques (56, 57) agencés sur la seconde surface principale (412, 422) de telle sorte qu'au niveau de la seconde surface principale la lumière est réfléchie par la seconde pluralité d'éléments prismatiques.

3. Dispositif électroluminescent selon l'une quelconque des revendications ci-dessus, dans lequel, pour chacune des au moins deux lamelles (41, 42), chacun des éléments prismatiques (53, 54, 55) de la première pluralité d'éléments prismatiques et de la seconde pluralité d'éléments prismatiques comprend une première facette (531, 541, 551) faisant face vers l'espacement et étant conçue pour collimater au moins une partie de la lumière incidente sur celle-ci et une seconde facette (532, 542, 552) faisant face vers l'espacement et étant conçue pour réfléchir la lumière incidente sur celle-ci vers la cavité.

4. Dispositif électroluminescent selon la revendication 3, dans lequel la première facette (531, 541, 551) comprend une première pente et la seconde facette (532, 542, 552) comprend une seconde pente, dans lequel la première pente comprend un premier angle de pente (α) par rapport à la première surface principale et la seconde pente comprend un second angle de pente (β) par rapport à la première surface principale, dans lequel le premier angle de pente (α) est compris entre 5° et 35°, et dans lequel le second angle de pente (β) est compris entre 90° et 55°.

5. Dispositif électroluminescent selon la revendication 4, dans lequel la somme du premier angle de pente (α) et du second angle de pente (β) est comprise entre 80° et 110°, ou dans lequel la somme du premier angle de pente (α) et du second angle de pente (β) est de 90°.

6. Dispositif électroluminescent selon la revendication 4 ou 5, dans lequel :
le premier angle de pente (α) est de 5° et le second angle de pente (β) est de 85°, ou
le premier angle de pente (α) est de 10° et le second angle de pente (β) est de 80°, ou
le premier angle de pente (α) est de 15° et le second angle de pente (β) est de 75°, ou
le premier angle de pente (α) est de 20° et le second angle de pente (β) est de 70°, ou
le premier angle de pente (α) est de 25° et le second angle de pente (β) est de 65°, ou
le premier angle de pente (α) est de 30° et le second angle de pente (β) est de 60°, ou
le premier angle de pente (α) est de 35° et le second angle de pente (β) est de 55°, ou
le premier angle de pente (α) est de 20° et le second angle de pente (β) est de 60°, ou
le premier angle de pente (α) est de 20° et le second angle de pente (β) est de 80°, ou
le premier angle de pente (α) est de 20° et le second angle de pente (β) est de 90°.

7. Dispositif électroluminescent selon l'une quelconque des revendications 3 à 6, dans lequel la première facette et la seconde facette s'étendent selon un angle (γ) compris entre 70° et 100° l'une par rapport à l'autre ou selon un angle (γ) de 90° l'une par rapport à l'autre.

8. Dispositif électroluminescent selon l'une quelconque des revendications ci-dessus, dans lequel chacun des éléments prismatiques (53, 54, 55) d'au moins l'une de la première pluralité d'éléments prismatiques et de la seconde pluralité d'éléments prismatiques comprend une ou plusieurs d'une largeur (W) étant inférieure ou égale à 1 mm et d'une hauteur (H) étant inférieure ou égale à 0,3 mm.

9. Dispositif électroluminescent selon l'une quelconque des revendications ci-dessus, dans lequel chacune des au moins deux lamelles (41, 42) comprend une longueur (L) d'au moins 10 mm.

10. Dispositif électroluminescent selon l'une quelconque des revendications ci-dessus, dans lequel la largeur (S) de l'espacement (9) entre les au moins deux lamelles est d'au moins 2 mm, la largeur (S) de l'espacement étant définie comme la distance la plus courte entre les premières surfaces principales (411, 421) opposées des lamelles (41, 42) respectives.

11. Dispositif électroluminescent selon l'une quelconque des revendications ci-dessus, dans lequel au moins l'une de la cavité (3), la première pluralité d'éléments prismatiques (51) et la seconde pluralité d'éléments prismatiques (52) comprennent une réflectivité d'au moins 90 % pour la lumière d'une longueur d'onde dans la plage de 280 nm à 400 nm.

12. Dispositif électroluminescent selon l'une quelconque des revendications 4 à 10 ci-dessus, dans lequel chacun des éléments prismatiques (53, 54, 55) de la première pluralité d'éléments prismatiques (51) et de la seconde pluralité d'éléments prismatiques (52) comprend une hauteur (H) s'étendant dans une direction perpendiculaire à la première surface principale (411), et dans lequel au moins l'un du premier angle de pente (α), du second angle de pente (β) et de la hauteur (H) d'un élément prismatique donné varie en fonction de la position de cet élément prismatique.

13. Dispositif électroluminescent selon la revendication 12, dans lequel au moins l'un du premier angle de pente (α) et du second angle de pente (β) augmente avec la distance par rapport à la cavité.

14. Dispositif électroluminescent selon l'une quelconque des revendications ci-dessus dans lequel la source lumineuse est conçue pour émettre une lumière UV, en fonctionnement, ayant une distribution spectrale avec un pic d'émission le plus élevé dans la plage de longueurs d'onde de 100 nm - 400 nm.

15. Luminaire ou lampe comprenant un dispositif électroluminescent selon l'une quelconque des revendications ci-dessus.
